# EUROPEAN PATENT APPLICATION

(11) **EP 1 655 286 A1**
(43) Date of publication of application: **10.05.2006**
(21) Application number: 04746514.1
(22) Date of filing: 21.06.2004
(51) Int. Cl.: C07D 213/56, B01D 9/02, A61K 31/4402, A61P 9/06

(54) **PROCESS FOR PRODUCING LOW-MELTING CRYSTAL OF FREE DISOPYRAMIDE BASE**

(30) Priority: 08.08.2003 JP 2003206904
(71) Applicant: Sumitomo Chemical Company, Limited, Tokyo 104-8260 (JP)
(72) Inventor: YAMAMOTO, Tomiaki, Sumitomo Chemical Company, Ltd, Anpachi-gun, Gifu 5030125 (JP); INOUE, Haruyuki, Sumitomo Chemical Company, Ltd, Anpachi-gun, Gifu 5030125 (JP)
(74) Representative: Vossius & Partner
(86) International application number: PCT/JP2004/009045
(87) International publication number: WO 2005/014548

(57) **Abstract**

The present invention provides a production method of a low melting point type crystal of disopyramide free base, which comprises a step of dissolving disopyramide free base in a solvent, a step of crystallizing the disopyramide free base from the obtained solution and a step of aging the crystal of the disopyramide free base, wherein the solvent is a mixed solvent of toluene and one or two kinds of hydrocarbon solvents selected from heptane and hexane. According to the present invention, a method capable of stably producing a low melting point type crystal of disopyramide free base on an industrial scale in a high yield can be provided.

## Description

### Technical Field

The present invention relates to a production method of a low melting point type crystal of disopyramide free base useful as a therapeutic agent for arrhythmia.

### Background Art

Disopyramide [α-(2-diisopropylaminoethyl)-α-phenyl-2-pyridineacetamide] used as a therapeutic agent for arrhythmia is in the form of a free base or a phosphate. Of these, disopyramide free base has a polymorphic crystal and comprises two crystal forms; a low melting point type crystal (85 to 87°C) and a high melting point type crystal (95 to 98°C) (pharmaceutical product Interview Form "Rythmodan capsule", rev. March, 2003, p. 4). From the viewpoint of pharmaceutical dissolution properties and the like, the low melting point type crystal is considered to be preferable. Of the two crystal forms, the high melting point type crystal is thermodynamically stable, and the low melting point type crystal requires strict control during production and as a pharmaceutical preparation because it easily converts to a high melting point type crystal.

### Disclosure of the Invention

There have heretofore been reported methods of obtaining a crystal of disopyramide free base (see US Patent No. 3225054 and JP-A-56-139461). For example, US Patent No. 3225054 discloses a method of crystallizing disopyramide free base from hexane. However, the obtained crystal has a melting point of 94.5 to 95°C, which is of a high melting point type crystal.

Moreover, JP-A-56-139461 discloses a method of crystallizing disopyramide free base from a 95:5 mixed solvent of hexane-ethanol. This method affords a low melting point type crystal having a melting point of 84 to 86°C. However, since the yield is as low as 51 to 59%, this method cannot be said to be advantageous.

It is therefore an object of the present invention to provide a method of stably producing a low melting point type crystal of disopyramide free base on an industrial scale and in a high yield.

The present inventors have conducted intensive studies in an attempt to achieve the above-mentioned object. As a result, they have found that a low melting point type crystal of disopyramide free base can be stably obtained by crystallizing the free base from a mixed solvent system of hydrocarbon solvent and toluene.

Moreover, they have found that the crystal does not convert to a high melting point type crystal by maintaining the drying temperature at not more than a predetermined level, which resulted in the completion of the present invention. Accordingly, the present invention affords the following.
(1) A production method of a low melting point type crystal of disopyramide free base, which comprises a step of dissolving disopyramide free base in a solvent (hereinafter to be also referred to as a dissolution step), a step of crystallizing the disopyramide free base from the obtained solution (hereinafter to be also referred to as a crystallization step) and a step of aging the crystal of the disopyramide free base (hereinafter to be also referred to as an aging step), wherein the solvent is a mixed solvent of toluene and one or two kinds of hydrocarbon solvents selected from heptane and hexane.
(2) The production method of the above-mentioned (1), wherein the ratio of the hydrocarbon solvent and toluene (hydrocarbon solvent:toluene) of the mixed solvent is 4:1 to 7.5:1 by volume ratio.
(3) The production method of the above-mentioned (1) or (2), wherein the amount of the mixed solvent of the hydrocarbon solvent and toluene is 4 ml to 4.5 ml per 1 g of the disopyramide free base.
(4) The production method of any of the above-mentioned (1) to (3), wherein a stirring rate during the crystallization step is 4 m/s to 6 m/s at the tip of a stirring blade.
(5) The production method of any of the above-mentioned (1) to (4), wherein a stirring rate during the aging step is 2 m/s to 3 m/s at the tip of a stirring blade.
(6) The production method of any of the above-mentioned (1) to (5), further comprising a step of drying the crystal of the disopyramide free base after crystallization and aging at not more than 60°C (hereinafter to be also referred to as a drying step).

### Detailed Description of the Invention

The production method of a low melting point type crystal of disopyramide free base of the present invention (hereinafter to be simply referred to as the production method of the present invention) is described in detail in the following.

The production method of the present invention comprises
(a) a step of dissolving (dissolution step) disopyramide free base in a mixed solvent of toluene and one or two kinds of hydrocarbon solvents selected from heptane and hexane,
(b) a step of crystallizing (crystallization step) the disopyramide free base from the solution obtained in the dissolution step, and
(c) a step of aging (aging step) the crystal of the disopyramide free base after the crystallization step, which may further comprise
(d) a step of drying (drying step) the crystal of the disopyramide free base after crystallization and aging at not more than 60°C.

By these steps, a low melting point type crystal of disopyramidefree base can be stably obtained on an industrial scale in a high yield.

Each step is explained in detail in the following.

### (a) Dissolution step

The disopyramide free base to be used for the dissolution step can be a crystal produced by a known method (e.g., method described in the above-mentioned patent reference 1). Alternatively, disopyramide phosphate used as a pharmaceutical product may be treated with a base (e.g., sodium hydroxide, potassium hydroxide etc.) to give a free base to be used for the dissolution step.

The crystal form of disopyramide free base to be used for the dissolution step is not particularly limited and may be any. However, a high melting point type crystal, or a mixture of a high melting point type crystal and a low melting point type crystal is preferable.

The hydrocarbon solvent to be used for the dissolution step may be heptane or hexane alone, or a mixture of these. When they are to be mixed, they can be mixed at any ratio.

When disopyramide free base is to be dissolved in a mixed solvent of a hydrocarbon solvent and toluene, disopyramide free base may be dissolved in a mixture of a hydrocarbon solvent and toluene, or the free base may be dissolved in one of the solvents and then the other solvent may be added. When the other solvent is to be added after dissolution in one of the solvents, it is preferable to add a hydrocarbon solvent after dissolution in toluene having a high solubility.

Moreover, when disopyramide free base obtained by treating disopyramide phosphate with a base is to be used, a solution obtained by treating disopyramide phosphate with an aqueous alkaline solution (e.g., aqueous sodium hydroxide solution, aqueous potassium hydroxide solution etc.) and extracting the free disopyramide with a mixed solvent of a hydrocarbon solvent and toluene may be used.

In the mixed solvent of a hydrocarbon solvent and toluene, the ratio of the hydrocarbon solvent and toluene (hydrocarbon solvent:toluene) is 4:1 to 7.5:1, preferably 4.7:1 to 6.3:1, by volume ratio. When the volume ratio is smaller than 4:1, the yield may decrease, and when the volume ratio is greater than 7.5:1, crystallization may not proceed or a high melting point type needle crystal may be precipitated.

The amount of the mixed solvent of a hydrocarbon solvent and toluene is 4 ml to 4.5 ml, preferably 4.2 ml to 4.3 ml, per 1 g of the disopyramide free base. When the amount of the mixed solvent is less than 4 ml per 1 g of the disopyramide free base, the disopyramide free base may not be completely dissolved and when it is greater than 4.5 ml, the yield may decrease.

The dissolution temperature during the dissolution step is not particularly limited as long as it permits sufficient dissolution of a crystal of disopyramide free base, and is generally 60 to 80°C.

The solution obtained in the dissolution step may be subjected as it is to the next crystallization step. In addition, it may be subjected to the crystallization step after hot filtration to remove impurities.

### (b) Crystallization step

In the production method of the present invention, the crystallization step covers from the start of the precipitation of a crystal from the solution of the disopyramide free base obtained in the dissolution step to almost the completion of the precipitation.

To be specific, when the temperature (e.g., 60 to 80°C) at which the disopyramide free base was dissolved in a mixed solvent of a hydrocarbon solvent and toluene in the dissolution step is gradually lowered, the crystal begins to precipitate (about 38 to 40°C), and the temperature starts to rise due to the heat of crystallization, and when the precipitation of the crystal almost ends, generation of the heat of crystallization ceases and the temperature rise stops.

Therefore, by monitoring the temperature of the system, the time point when the temperature rise due to the heat of crystallization stops can be taken as the end point of the crystallization step.

Since the temperature that increases due to the heat of crystallization varies depending on the scale, shape of container and the like, the end point can be appropriately determined by monitoring the temperature of the system.

In the crystallization step, a low melting point type seed crystal is preferably added before crystal precipitation to afford a specific crystal having a low melting point.

The amount of the seed crystal only needs to be 5 to 20 mg per 100 g of the disopyramide free base which is the starting material.

The temperature of addition of the seed crystal is preferably immediately before the start of crystal precipitation, which is specifically 35 to 55°C, preferably 45 to 50°C.

The crystallization step is preferably performed under stirring for smooth precipitation of crystal, where the stirring rate is preferably 4 m/s to 6 m/s, more preferably 4.5 m/s to 5.5 m/s, at the tip of the stirring blade. When the rate at the tip of the stirring blade is slower than 4 m/s, a high melting point type crystal tends to precipitate, and when it is faster than 6 m/s, stirring becomes overloaded, and the impeller and motor may get broken.

The time of the crystallization step can be appropriately determined while monitoring the temperature of the system, because the temperature rise rate due to the heat of crystallization varies depending on the scale, shape of container, stirring rate and the like.

### (c) Aging step

The aging step in the production method of the present invention increases, after the crystallization step, the yield and affords a uniform and stable low melting point type crystal in the system.

To be specific, after cease of the temperature rise due to the heat of crystallization in the crystallization step, by stirring while gradually lowering the temperature, the yield can be increased and the low melting point type crystals in the system can be made uniform.

The aging time is not particularly limited but it is preferably 2 hrs to 5 hrs, more preferably 3 hrs to 4 hrs.

The cooling rate in the aging step is preferably stepwise cooling. For example, the temperature at which increase due to the heat of crystallization stops is lowered to 35°C over 40 to 80 min, then from 35°C to 25°C over 40 to 80 min, and then from 25°C to 20°C over 80 to 120 min.

The stirring rate in the aging step is preferably 2 m/s to 3 m/s, more preferably 2.5 m/s to 2.7 m/s, at the tip of the stirring blade. When the rate at the tip of the stirring blade is faster than 3 m/s, the crystal easily converts to a high melting point type crystal, and when it is slower than 2 m/s, the crystal tends to form sediment.

The crystal thus crystallized and aged is filtered and washed to isolate a low melting point type crystal of disopyramide free base.

The filtration method may be a known method, which may be any method such as natural filtration, filtration under reduced pressure, pressure filtration, centrifugal filtration, press filtration and the like, and a method suitable for scale and the like can be appropriately selected.

As the solvent used for washing, a hydrocarbon solvent or a mixed solvent of a hydrocarbon solvent and toluene is preferable, and from the aspect of yield, use of a hydrocarbon solvent alone is more preferable.

While the stirring blade to be used for stirring in the production method of the present invention is not particularly limited as long as it is generally used for a stirrer, specific examples thereof include pitched paddle blade, flat paddle blade, propeller blade, anchor impeller, Pfaudler blade, turbine impeller, BRUMARGIN blade, MAX BLEND impeller (manufactured by Sumitomo Heavy Industries, Ltd.), FULLZONE impeller (manufactured by Shinko Pantec Co., Ltd.), ribbon impeller, SUPERMIX impeller (manufactured by Satake Chemical Equipment Mfg., Ltd.) and the like can be mentioned, with preference given to propeller blade, anchor impeller, Pfaudler blade and the like.

The rate at the tip of the stirring blade can be calculated from the following formula wherein π is circular constant, ϕ is diameter (m) of the stirring blade, and n is rps. rate at the tip of the stirring blade (m/s) =π×ϕ×n

### (d) Drying step

The low melting point type crystal of disopyramide free base isolated as mentioned above can be dried by known drying method, such as air drying, ventilation drying, vacuum drying and the like, with preference given to vacuum drying.

For vacuum drying, the reduced pressure is preferably within the range of 1.3 kPa to 4 kPa. When the reduced pressure is higher than 4 kPa, the drying rate tends to be late and when it is lower than 1.3 kPa, a pressure resistant container becomes necessary, which tends to prove industrially disadvantageous.

When the drying temperature is high in the drying step, a low melting point type crystal easily converts to a high melting point type crystal. Therefore, drying at not more than 65°C is preferable, and drying at not more than 60°C is more preferable. While the lower limit of the drying temperature is not particularly limited, drying at not less than 40°C is preferable to prevent low drying rate.

### Examples

The present invention is explained in detail by referring to Examples, which are not to be construed as limitative.

### Example 1

Disopyramide free base (40 g) was added to a mixed solvent of n-heptane (140 ml) and toluene (35 ml), and the mixture was dissolved by heating to 65°C. The mixture was allowed to cool while stirring at a rate at the tip of the stirring blade of 5.18 m/s, and when it reached 45°C, a small amount of a low melting point type crystal of disopyramide free base was added as a seed crystal to allow crystallization. The temperature of the system was monitored and when the temperature rise due to the heat of crystallization ended (46°C), the rate at the tip of the stirring blade was decreased to 2.59 m/s and the mixture was aged by cooling to 20°C over 3 hr 20 min. The crystal was collected by filtration, washed with n-heptane (10 ml) and dried under reduced pressure (3 kPa) at 60°C for 5 hrs to give a crystal (35.4 g) of disopyramide free base. yield: 88.5%, melting point: 86.1°C

### Example 2

Disopyramide free base (40 g) was added to a mixed solvent of n-heptane (140 ml) and toluene (22.4 ml), and the mixture was dissolved by heating to 67°C. The mixture was allowed to cool while stirring at a rate at the tip of the stirring blade of 5.2 m/s, and when it reached ca. 45°C, a small amount of a low melting point type crystal of disopyramide free base was added as a seed crystal to allow crystallization. The temperature of the system was monitored and when the temperature rise due to the heat of crystallization ended (46°C), the rate at the tip of the stirring blade was decreased to 2.59 m/s and the mixture was aged by cooling to 20°C over 3 hr 20 min. The crystal was collected by filtration, washed with n-heptane (10 ml) and dried under reduced pressure (3 kPa) at 60°C for 5 hrs to give a crystal (37.0 g) of disopyramide free base. yield: 92.5%, melting point: 85.9°C

### Example 3

n-Heptane (600 ml) and toluene (156 ml) were added to disopyramide phosphate (271.5 g) dissolved in water (1000 ml). A 25% aqueous sodium hydroxide solution (250 ml) was added dropwise over 50 min at 23 to 27°C. The pH of the aqueous layer then was 12.65. After partitioning, the organic layer was heated to 67°C, and washed twice with 250 ml of warm water. Radiolite (10 g) was added to the organic layer and the mixture was filtered and washed with n-heptane (137.5 ml). The filtrate was cooled while stirring at a rate at the tip of the stirring blade of 5.2 m/s, and when it reached 45°C, a low melting point type crystal of disopyramide free base was added as a seed crystal to allow crystallization. The temperature of the system was monitored and when the temperature rise due to the heat of crystallization ended (46°C), the rate at the tip of the stirring blade was decreased to ca. 2.6 m/s and the mixture was aged by cooling to 20°C over 4 hr. The crystal was collected by filtration, washed with a mixed solvent of n-heptane (100 ml) and toluene (17.5 ml) and dried under reduced pressure (3 kPa) at 60°C for 7 hrs to give a crystal (197.4 g) of disopyramide free base. yield: 93.7%, melting point: 85.7°C

### Example 4

In the same manner as in Example 2 except n-hexane (140 ml) was used instead of n-heptane, a crystal (37.2 g) of disopyramide free base was obtained. yield: 93.0%, melting point: 85.9°C

### Comparative Example 1

The low melting point type crystal of the disopyramide free base obtained in Example 3 was further dried at 70°C for 7 hrs. The obtained crystal had a melting point of 96.3°C and the crystal had converted to a high melting point type crystal.

### Comparative Example 2

The recrystallization described in Example 2 or 3 of JP-A-56-139461 was reproduced. The yield was about 82%, stable production of a low melting point type crystal of disopyramide free base was unattainable since a high melting point type crystal and a low melting point type crystal of disopyramide free base were sometimes obtained.

### Industrial Applicability

According to the production method of the present invention, a method capable of stably producing a low melting point type crystal of disopyramide free base on an industrial scale in a high yield can be provided.

This application is based on patent application No. 2003-206904 filed in Japan, the contents of which are hereby incorporated by reference.

## Claims

1. A production method of a low melting point type crystal of disopyramide free base, which comprises a step of dissolving disopyramide free base in a solvent, a step of crystallizing the disopyramide free base from the obtained solution and a step of aging the crystal of the disopyramide free base, wherein the solvent is a mixed solvent of toluene and one or two kinds of hydrocarbon solvents selected from heptane and hexane.

2. The production method of claim 1, wherein the ratio of the hydrocarbon solvent and toluene (hydrocarbon solvent:toluene) of the mixed solvent is 4:1 to 7.5:1 by volume ratio.

3. The production method of claim 1 or 2, wherein the amount of the mixed solvent of the hydrocarbon solvent and toluene is 4 ml to 4.5 ml per 1 g of the disopyramide free base.

4. The production method of any of claims 1 to 3, wherein a stirring rate during the step of crystallizing is 4 m/s to 6 m/s at the tip of a stirring blade.

5. The production method of any of claims 1 to 4, wherein a stirring rate during the step of aging is 2 m/s to 3 m/s at the tip of a stirring blade.

6. The production method of any of claims 1 to 5, further comprising a step of drying the crystal of the disopyramide free base after crystallization and aging at not more than 60°C.
